# EUROPEAN PATENT SPECIFICATION

(11) **EP 2 076 140 B1**
(45) Date of publication and mention of the grant of the patent: **02.01.2013**
(21) Application number: 07805721.3
(22) Date of filing: 18.07.2007
(51) Int. Cl.: A61K 35/20, A61K 38/00, A23C 9/142, C07K 14/79, A61K 38/40

(54) **PRODUCT, SUITABLE TO BE GIVEN TO THE HUMAN BODY FOR ITS PROTECTIVE EFFECTS AND HEALTH BENEFITS, BASED ON COW BUFFALO'S MILK SELF-ENRICHED WITH LACTOFERRIN, OR PURE LACTOFERRIN, OBTAINED THROUGH MEMBRANE SEPARATIVE PROCESSES**
PRODUKT, DAS SICH FÜR DIE ABGABE AN DEN MENSCHLICHEN KÖRPER ZUR ENTFALTUNG SEINER SCHUTZWIRKUNGEN UND GESUNDHEITSFÖRDERNDEN WIRKUNGEN EIGNET, BASIEREND AUF BÜFFELKUHMILCH, DIE MIT EIGENLACTOFERRIN ODER MIT REINEM LACTOFERRIN, DAS DURCH MEMBRANTRENNUNGSVERFAHREN ERHALTEN WURDE, ANGEREICHERT IST
PRODUIT POUVANT ETRE DONNE A UN ORGANISME HUMAIN EN RAISON DE SES EFFETS PROTECTEURS ET DE SES AVANTAGES POUR LA SANTE, BASE SUR DU LAIT DE BUFFLESSE AUTO-ENRICHI EN LACTOFERRINE, OU LACTOFERRINE PURE OBTENUE PAR DES PROCEDES DE SEPARATION A TRAVERS UNE MEMBRANE

(30) Priority: 07.08.2006 IT RM20060434
(43) Date of publication of application: 08.07.2009
(73) Proprietor: Raimondi, Pietro, 00162 Roma (IT)
(72) Inventor: Raimondi, Pietro, 00162 Roma (IT)
(74) Representative: Zizzari, Massimo
(86) International application number: PCT/IT2007/000509
(87) International publication number: WO 2008/018103

(56) References cited:
- WO-A-03/073866
- JP-A- 7 300 425
- US-A- 5 919 913
- US-A1- 2005 197 495
- MAHESHWARI R K ET AL: "EFFECT OF HEATING AND FROZEN STORAGE ON THE LEVEL OF NATIVE, APO AND COBALT, COPPER, MANGANESE AND CHROMIUM COMPLEXED BUFFALO MILK LACTOFERRIN" MILCHWISSENSCHAFT, VV GMBH VOLKSWIRTSCHAFTLICHER VERLAG. MUNCHEN, DE, vol. 45, no. 4, 1990, pages 225-229, XP001024632 ISSN: 0026-3788
- INDYK H E ET AL: "Determination of lactoferrin in bovine milk, colostrum and infant formulas by optical biosensor analysis" INTERNATIONAL DAIRY JOURNAL, ELSEVIER APPLIED SCIENCE, BARKING,, GB, vol. 15, no. 5, May 2005 (2005-05), pages 429-438, XP004820390 ISSN: 0958-6946
- SHARMA A K ET AL: "Three-dimensional structure of mare diferric lactoferrin at 2.6 A resolution" JOURNAL OF MOLECULAR BIOLOGY, LONDON, GB, vol. 289, no. 2, 4 June 1999 (1999-06-04), pages 303-317, XP004462054 ISSN: 0022-2836
- DATABASE WPI Week 199849 Derwent Publications Ltd., London, GB; AN 1998-577005 XP002420284 & JP 10 259137 A (SNOW BRAND MILK PROD CO LTD) 29 September 1998 (1998-09-29)

## Description

### TECHNICAL FIELD

The present invention concerns a method addressed to production of a nutraceutical having a high content of lactoferrin that, if ingested, results particularly effective for a protective and benefit use respect to functions of the human body. This product is obtained starting from cow buffalo's milk through membrane separative processes.

In fact, it is known that milk and milk-dairy products are essential for the human nutrition. In the constituents of milk there are proteins, peptides, enzymes and other substances that are normally given from mother to child during the nursing period, and that are considered to be essential for health of the newborn baby.

One of these proteins, the lactoferrin, is actually subject of many studies, according to its important characteristic of being a iron-carrier from intestine to the blood haemoglobin and to its antimicrobial, antiviral, antinflammatory, and prebiotic action. Therefore, lactoferrin is a iron-carrier glycoprotein, belonging to the group of transferrins, and it is contained in the human milk. Furthermore, this molecule is also contained in milk of other mammals, like in example: cattles, pigs, buffalos, goats, rodents and equines.

The lactoferrin, besides the iron-carrier employed function, is a natural antioxidant and a powerful booster of *natural-killer* cells, it has an important role in the defence against tumours, it controls the granulepoiesis, the cellular cytotoxicity antibody- dependant, the production of cytokines, and the *in vitro* growth of some specific cells. It employes an additional function stimulating the immune defence system, it inhibits the *in vivo* and *in vitro* release of histamine from mastcells. The digestion with pepsin produces peptides, having antimicrobial action, so-called lactoferricin.

### BACKGROUND ART

In the prior art, some processes are well-known to isolate, from the cattle's milk, proteins from fat and lactose, and therefore to produce on a large industrial scale food supplements and/or pharmacological products.

Patent application US2005/197495 A1 (Naidu A.S.[US] Naidu A. Satyanarayan [US]) discloses milk, which is enriched with purified lactoferrin obtainable from dairy sources, by multi-stage purification involving precipitation, chromatography, dialysis, etc. The prior art teaches various techniques suitable for isolating lactoferrin from milk, like that disclosed in PCT application WO03/073866 A (Upfront Chromatography AS [DK]; Lihme Allan Otto Fog [DK]), regarding precipitation, extraction, etc., and that disclosed in patent JP 7 300425 A (Snow Brand Milk Prod Co Ltd) with reference to a method involving dialysis at a certain step.

Furthermore, considering the following document: Maheshwari, R. K. et al.: "Effect of heating and frozen storage on the level of native, apo and cobalt, copper, manganese and chromium complexed buffalo milk lactoferrin", Milchwissenschaft, VV Gmbh Volkswirtschaftlicher Verlag, Munchen, DE, Vol.45, n.4, 1990, pages 225-229, some technics are further described, like i.e. ion exchange, chromatography and gel filtration.

Basically, the actual products on the market belong to three main classes: whey proteins isolated through ionic exchange; concentrated whey proteins; and whey proteins isolated through microfiltration. For each class, an index of quality is defined, essentially in direct ratio to quantity of contained proteins, and in inverse ratio to quantity of fat and lactose content beyond a certain limit. Another important parameter is the cost for protein extraction that defines the final product's price to the market. Indeed, the highest quality products, having more than 90% of proteins and less than 1% of fat and lactose content, have either a typical high cost as drawback.

### DISCLOSURE OF INVENTION

The above situation leads to a method addressed to production of a nutraceutical product, subject of the present invention, that, besides the high protein content, particularly of lactoferrin, is characterized by a particularly simple, effective and cheap method of production.

Therefore, the main objective of the present invention, is to provide a method addressed to production of a nutritious drink, having some considerable organoleptic characteristics and a high level of lactoferrin, able to stimulate the immune defence system, to increase the absorption of iron through the intestine, and to represent an alternative to the cow's milk, because it doesn't create phoenomena of intolerance to food.

Another objective is to provide a method addressed to production of a nutritious drink having the additional characteristic of a lower fat content respect to the cow buffalo's milk and the same antioxidant power of the cow buffalo's milk.

A further objective is to provide a method suitable for large-scale industrial production, employing available and cheap components, characterized by reasonable time and costs, and limiting the waste of raw materials.

Therefore, it is specific subject of the present invention a method for extraction of lactoferrin from cow buffalo's milk through membrane separative processes, according to two ways of production:
1. cycles of dialysis with different external solutions, keeping the same membrane:
   wherein the starting solution is always the same inside the membrane and the
   external solution is renewed in each cycle of dialysis;
2. cycles of dialysis with different membranes, keeping the same external solution: wherein the internal solution, instead, changes time by time in the various cycles of dialysis.

### BRIEF DESCRIPTION OF DRAWINGS

The present invention will now be described for illustrative but not limitative purposes, according to its preferred embodiments, with particular reference to the figures of the enclosed drawings, wherein:
figure 1 is a schematic perspective view of a particular, concerning a semi-permeable membrane for dialysis;
figure 2 is a lateral sectioned view of a device for dialysis, enclosing a semi-permeable membrane allowing the transfer of particles from one solution to the other one;
figures 3, 4, 5 and 6 are respective lateral sectioned views of a device for dialysis, corresponding to a specific state, related to a first method of lactoferrin extraction;
figure 7 is a graphical view of lactoferrin concentration as a function of the number of cycles, related to a first method of lactoferrin extraction;
figure 8 is a schematic view representing the final phase of mixing, related to a first method of lactoferrin extraction;
figures 9, 10, 11 and 12 are respective lateral sectioned views of a device for dialysis, corresponding to a specific state, related to a second method of lactoferrin extraction;
figure 13 is a lateral sectioned view of a device in the final phase of mixing, related to a second method of lactoferrin extraction;
figure 14 is a graphical view of lactoferrin concentration as a function of the number of cycles, related to a second method of lactoferrin extraction.

### BEST MODES FOR CARRYING OUT THE INVENTION

It is herein underlined that, in the following, only some of the many conceivable embodiments of the present invention will be described, and they are just particular examples that do not introduce any limitations, having the possibility to describe many other embodiments based on the disclosed technical solution of the present invention. More in particular, two main methods for extraction of lactoferrin from cow buffalo's milk will be described, the same methods leading to the synthesis of products based on cow buffalo's milk enriched with lactoferrin, or pure lactoferrin. Both methods employ membrane separative processes and, more in particular, extraction methods through dialysis.
The dialysis is a process providing the separation of molecules according to their dimension, using semi-permeable membranes with pores having a smaller size than macromolecules. This concept has been useful to separate molecules with different dimension in the cow buffalo's whey, using a dialysis membrane having pores with a proper diameter.

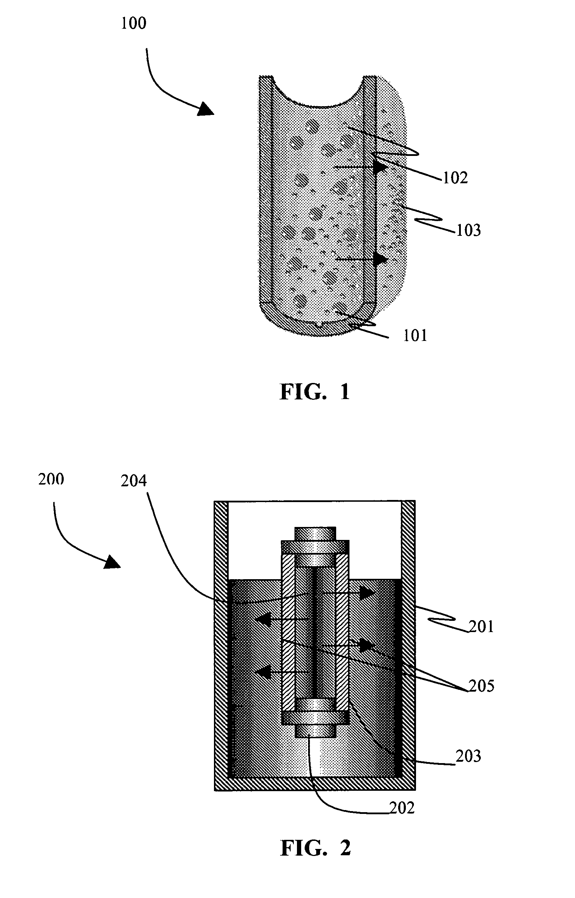

In figure 1, an example of cylindrical semi-permeable membrane 100 is illustrated. It allows some molecules 102, 103 to pass through and, at the same time, prevents other molecules 101 to do the same thing.
In figure 2, a typical example of a device for dialysis 200 is illustrated, with an external container 201 containing a first solution 202, and an internal container 203, containing a second solution 204. The internal container 203 includes a semi-permeable membrane 100, able to allow the transfer of some molecules from the second solution 204 to the first solution 202.
Both the processes for lactoferrin extraction include a previous phase where the fat content in the cow buffalo's milk is decreased. This is possible through a milk centrifuge process at 4000 rpm for 10 minutes. In such a way, the fat concentrates itself on the upper layer, where is easily removable using a paddle or a spoon.
This quantity of fat will be mixed again in a second time, in a specified measure, to weigh the final composition (it will correspond to 2-3% of the total, in order to keep organoleptic characteristics similar to the initial milk).
It is important to underline that the decreased quantity of fat does not change the antioxidant power of the cow buffalo's milk, because experiments proved that the main contribution to this parameter is essentially given by whey and not by fat content.

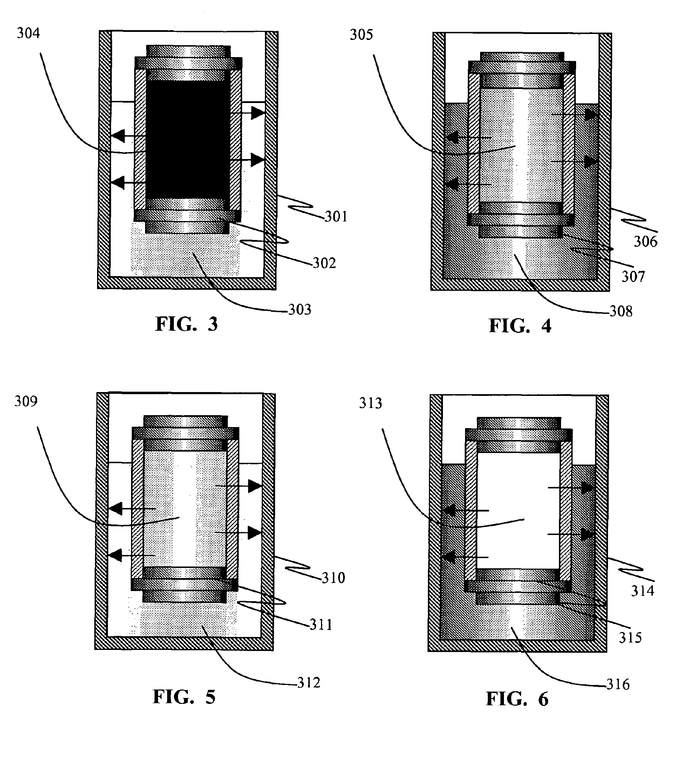

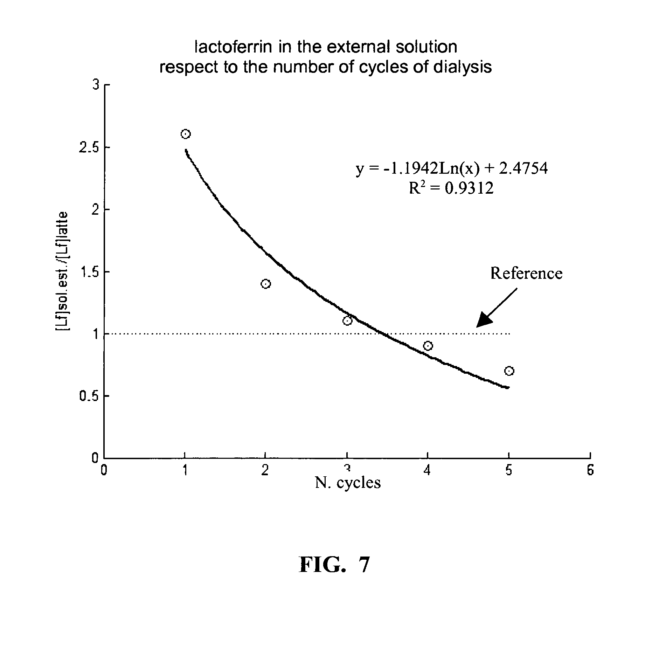

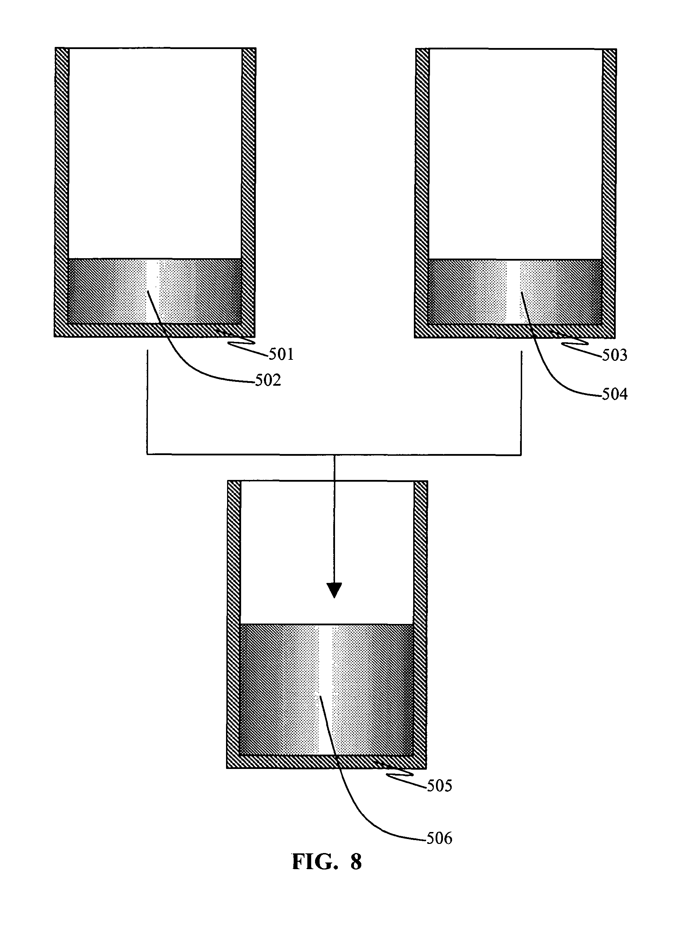

The methods for lactoferrin extraction of the prior art have some difficulties arising from the final use in food industry. In fact, normally the purification process for analytical purposes (HPLC analysis, electrophoresis, etc.) requires reactive precipitants and other materials that prevent any possible use for food. Therefore, the definition of an alternative extraction method is not trivial. One possibility is to make the cow buffalo's milk to evaporate at a temperature of 35°C, so that to avoid the denaturation of contained proteins, and at a lower pressure than the environmental one, in order to speed up the process. Unfortunately, this method requires a big quantity of time and energy.
Therefore, the method disclosed in the present invention employs a process of dialysis, wherein the molecules having a lower diameter than size of the semi-permeable membrane's pores transfer from the solution with high concentration to the solution with low concentration. More in particular, the membrane is made of cellulose acetate and the solution where lactoferrin concentrates contains bidistilled water.
In the different phases of this method, the main objective is to define the detailed optimal conditions of dialysis, in order to achieve as much as possible quantity of lactoferrin with the minimum quantity of required time and energy.
Therefore, two possible ways have been found:
1. cycles of dialysis with external solution renewed at each cycle, keeping the same membrane;
2. cycles of dialysis with different membranes, keeping the same external solution for all the time long.
In a preferred embodiment of the present invention, related to the first case illustrated in figures 3, 4, 5 and 6, the starting solution is always the same inside the membrane and the external solution is renewed at each cycle of dialysis. In the first cycle - figure 3 - one litre of cow buffalo's milk 304 is inserted inside a dialysis tube 302 and is put in communication with 50 mLitres of bidistilled water 303 at 4°C for 30 minutes. At the end of 30 minutes - figure 4 - the external solution 308 will contain a certain quantity of lactoferrin and diffused salts and can be exchanged - figure 5 - with a new external solution containing other 50 mLitres of bidistilled water 312. At this point, a second cycle of dialysis - figure 6 - will allow the diffusion of an additional quantity of lactoferrin from the internal solution 313 to the external solution 316. In principle, the cycles of dialysis could follow each other for an indefinite time, according to the indicated scheme. However, at each cycle the quantity of extracted lactoferrin decreases logarithmically, following the graphical shape of figure 7. It is clear that the significant quantities of lactoferrin are obtained especially in the first two cycles, with respective values equal to 2.6 and 1.3 times the initial concentration in milk. This information suggests to use just the first two cycles of dialysis and to mix - figure 8 - the respective 50 mLitres of external solutions 502 and 504 in order to obtain 100 mLitres of solution 506 containing approximately two times the quantity of lattoferrin in the initial milk.
Then, the so obtained solution 506 is mixed with new cow buffalo's milk, where the fat has been previously removed, and finally the resulting solution is reintegrated with fat, up to a quantity nearly to 2-3%.
In such a way a product has been obtained, that is cow buffalo's milk enriched with lactoferrin and having a quantity of fat extremely lower than the initial milk. The same method can be employed to realize different types of food supplements containing high quantities of lactoferrin.

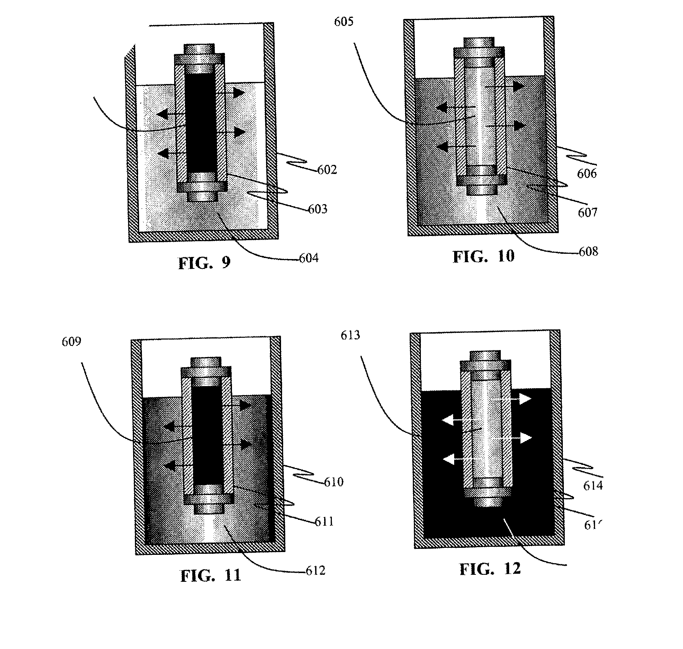

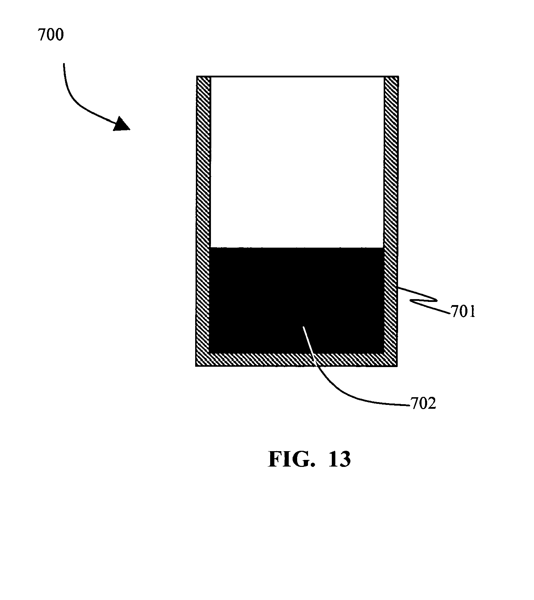

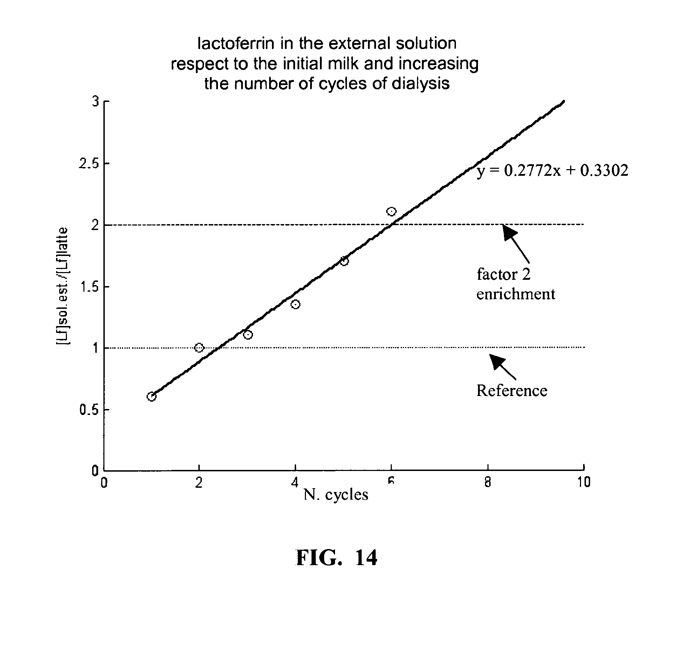

In case it is required a higher level of enrichment in the previous solution, an alternative method is that illustrated in figures 9, 10, 11 and 12, related to a second preferred embodiment of the present invention, where, in the different cycles of dialysis, the external solution is kept always the same, and the internal solution changes from time to time. In such a way, the necessary time for lactoferrin extraction is longer respect to the previous method, but the consumption of raw material is approximately 50% less, and it is possible to achieve a higher level of enrichment respect to the previous method.
In the first cycle - figure 9 - 100 mLitres of cow buffalo's milk 601, with previously fat content removed, through a centrifuge process at 4000 rpm for 10 minutes, are inserted inside a dialysis tube 603 and are put in communication with 100 mLitres of bidistilled water 604 at 4°C for 30 minutes. At the end of 30 minutes - figure 10 - the external solution 608 will contain a certain quantity of lactoferrin and diffused salts. For cycles following the first one - figure 11 - the external solution is kept the same, and the dialysis membrane 607 is changed with a new one 611, containing 100 mLitres of new milk with previosly removed fat. This process is repeated for 6 different cycles, measuring from time to time the quantity of lactoferrin contained in the external solution. The quantity of extracted lactoferrin increases linearly for each following cycle, according to the graphical shape of figure 14, up to obtain a solution having a high concentration like the one illustrated in figure 13.

The described method requires 6 cycles to enrich the external solution up to factor 2, requiring 3 hours respect to just 1 hour of the previous method, and with a raw material consumption equal to 600 mLitres of milk. Therefore, the first way allows to achieve quick results, but requiring a higher consumption of raw material, instead the second way allows a limitation of waste and the achievement of a higher level of lactoferrin enrichment in the external solution, but it requires a longer time for process.

Therefore, all the above examples show that the present invention achieves the proposed objectives. In particular, it provides a method addressed to production of a nutritious drink, having some considerable organoleptic characteristics and a high level of lactoferrin, able to stimulate the immune defence system, to increase the absorption of iron through the intestine, and to represent an alternative to the cow's milk, because it doesn't create phoenomena of intolerance to food.

Then, the present invention provides a method addressed to production of a nutritious drink having the additional characteristic of a lower fat content respect to the cow buffalo's milk and the same antioxidant power of the cow buffalo's milk.

Furthermore, the present invention provides a method suitable for large-scale industrial production, employing available and cheap components, characterized by reasonable time and costs, and limiting the waste of raw materials.

The present invention has been described for illustrative but not limitative purposes, according to its preferred embodiments, but it is clear that modifications and/or changes can be introduced by those skilled in the art without departing from the relevant scope, as defined in the enclosed claims.

## Claims

1. Method for extraction of lactoferrin from cow buffalo's milk **characterized by** the following steps:
- in a first cycle, a definited quantity of cow buffalo's milk is inserted in a dialysis tube and put in communication with a definite quantity of bidistilled water at a definited temperature and for a definited quantity of time;
- at the end of the abovesaid quantity of time, a definited quantity of lactoferrin and diffused salts have diffused from said cow buffalo's milk to said bidistilled water, constituting outside a first solution concentrated of lactoferrin.

2. Method for extraction of lactoferrin from cow buffalo's milk, according to previous claim 1, **characterized by** the following additional steps:
- said first solution concentrated of lactoferrin is exchanged with a new and identical quantity of bidistilled water;
- in a second cycle, at a definited temperature and for a definited quantity of time, an additional quantity of lactoferrin and diffused salts have diffused from said cow buffalo's milk to said new bidistilled water, constituting outside a second solution concentrated of lactoferrin;
- a series of cycles, identical to the previous ones, follow each other for a definited number of times, up to achieve N solutions concentrated of lactoferrin obtained from the method (one for each cycle);
- the abovesaid N solutions concentrated of lactoferrin are mixed in a new container in order to obtain a unique final solution, having a higher level of lactoferrin concentration in respect of the initial concentration in the cow buffalo's milk,
so that it is possible to obtain a nutritious drink, having some considerable organoleptic characteristics and a high level of lactoferrin, having the same antioxidant power of the cow buffalo's milk, able to stimulate the immune defence system, to increase the absorption of iron through the intestine, and to represent an alternative to the cow's milk, because it doesn't create phoenomena of intolerance to food.

3. Method for extraction of lactoferrin from cow buffalo's milk, according to previous claim 2, **characterized by** the following additional steps:
- a new definited quantity of cow buffalo's milk is submitted to a centrifuge process and the upper layer of fat content is removed;
- said new definited quantity of cow buffalo's milk with fat content removed and said unique final solution are mixed in a new container in order to obtain cow buffalo's milk self-enriched with lactoferrin;
- a definited quantity of fat is mixed again with said cow buffalo's milk self-enriched with lactoferrin,
so that it is possible to obtain a nutritious drink, like the above described one, having the additional characteristic of a lower fat content in respect of the cow buffalo's milk.

4. Method for extraction of lactoferrin from cow buffalo's milk, according to one or more of the previous claims, **characterized in that** said number of cycles of dialysis N is equal to 2.

5. Method for extraction of lactoferrin from cow buffalo's milk, according to one or more of the previous claims, **characterized in that** said definited quantity of cow buffalo's milk is equal to 1 Litre.

6. Method for extraction of lactoferrin from cow buffalo's milk, according to one or more of the previous claims, **characterized in that** said definite quantity of bidistilled water is equal to 50 mLitres.

7. Method for extraction of lactoferrin from cow buffalo's milk, according to previous claim 1, **characterized in that**:
- said definited quantity of cow buffalo's milk has been previously submitted to a centrifuge process and the upper layer of fat content has been removed;
and **characterized by** the following additional steps:
- after said first solution concentrated of lactoferrin is formed, said dialysis tube is exchanged with a new one, containing an identical quantity of cow buffalo's milk with fat content removed;
- a series of cycles, identical to the previous ones, follow each other for a definited number of times, up to achieve a solution obtained from the method, having a level N (number of cycles) of concentration of lactoferrin;
so that it is possible to obtain a nutritious drink, having some considerable organoleptic characteristics and a high level of lactoferrin, having the same antioxidant power of the cow buffalo's milk, able to stimulate the immune defence system, to increase the absorption of iron through the intestine, and to represent an alternative to the cow's milk, because it doesn't create phoenomena of intolerance to food.

8. Method for extraction of lactoferrin from cow buffalo's milk, according to previous claim 7, **characterized in that**:
- a new definited quantity of cow buffalo's milk is submitted to a centrifuge process and the upper layer of fat content is removed;
- said new definited quantity of cow buffalo's milk with fat content removed and said solution obtained from the method are mixed in a new container in order to obtain cow buffalo's milk self-enriched with lactoferrin;
- a definited quantity of fat is mixed again with said cow buffalo's milk self-enriched with lactoferrin,
so that it is possible to obtain a nutritious drink, like the above described one, having the additional characteristic of a lower fat content in respect of the cow buffalo's milk.

9. Method for extraction of lactoferrin from cow buffalo's milk, according to one or more of the previous claims, **characterized in that** said dialysis tube has a semi-permeable membrane composed of cellulose acetate.

10. Method for extraction of lactoferrin from cow buffalo's milk, according to one or more of the previous claims from 7 to 9, **characterized in that** said number of cycles of dialysis N is equal to 6.

11. Method for extraction of lactoferrin from cow buffalo's milk, according to one or more of the previous claims from 7 to 10, **characterized in that** said definited quantity of cow buffalo's milk is equal to 100 mLitres.

12. Method for extraction of lactoferrin from cow buffalo's milk, according to one or more of the previous claims from 7 to 11, **characterized in that** said definite quantity of bidistilled water is equal to 100 mLitres.

13. Method for extraction of lactoferrin from cow buffalo's milk, according to one or more of the previous claims, **characterized in that** said definited temperature is equal to 4°C and that definited quantity of time is equal to 30 minutes.

14. Method for extraction of lactoferrin from cow buffalo's milk, according to previous claim 3 or previous claim 8, **characterized in that** said definited quantity of fat is equal to 2-3%.

15. Method for extraction of lactoferrin from cow buffalo's milk, according to previous claim 3 or previous claim 8, **characterized in that** said centrifuge process is realized at 4000 rpm for 10 minutes.

## Patentansprüche

1. Verfahren zur Extraktion von Lactoferrin aus Büffelmilch, das durch folgende Schritte gekennzeichnet ist:
- in einem ersten Zyklus wird eine definierte Menge an Büffelmilch ins Innere eines Dialyseschlauchs gegeben, der mit einer definierten Menge an bidestilliertem Wasser zu einer definierten Temperatur und für einen definierten Zeitintervall in Kontakt gebracht wird;
- am Ende des besagten Zeitintervalls werden eine definierte Menge an Lactoferrin und eine definierte Menge an Salzen von besagter Menge an Büffelmilch an besagte definierte Menge an bidestilliertem Wasser diffundiert und bildet so Außen eine erste konzentrierte Lösung Lactoferrin.

2. Verfahren zur Extraktion von Lactoferrin aus Büffelmilch, gemäß dem vorherigen Anspruch 1, das durch die folgenden zusätzlichen Schritte gekennzeichnet ist:
- besagte erste konzentrierte Lösung Lactoferrin wird durch neues bidestilliertes Wasser mit der gleichen Menge ersetzt;
- in einem zweiten Zyklus wird zu einer definierten Temperatur und für einen definierten Zeitintervall eine weitere Menge Lactoferrin und Salze aus besagter Menge an Büffelmilch zu besagter Menge an bidestilliertem Wasser diffundiert und bildet so Außen eine zweite konzentrierte Lösung Lactoferrin;
- eine Reihe von Zyklen, die mit den vorherigen identisch sind, folgen in einer endlichen Anzahl aufeinander, bis N konzentrierte Lösungen an Lactoferrin gewonnen werden, die aus dem Verfahren (eine für jeden Zyklus) gewonnen werden;
- die obengenannten N konzentrierten Lösungen Lactoferrin werden in einem Behälter vermischt, um eine einzige endgültige Lösung zu erhalten mit einer Konzentration an Lactoferrin, die größer ist, als die anfängliche Konzentration in der Büffelmilch,
so dass aus den bemerkenswerten organoleptischen Eigenschaften und mit einem hohen Gehalt an Lactoferrin mit derselben antioxidativen Kraft von Büffelmilch ein nahrhaftes Getränk gewonnen wird, das daher in der Lage ist, die Immunabwehr zu stimulieren, die Eisenaufnahme durch den Darm zu erhöhen und eine Alternative zu Kuhmilch zu stellen, da es keine Erscheinungen der Nahrungsmittelunverträglichkeit erzeugt.

3. Verfahren zur Extraktion von Lactoferrin aus Büffelmilch, gemäß dem vorherigen Anspruch 2, das durch die folgenden zusätzlichen Schritte gekennzeichnet ist:
- eine neue vordefinierte Menge an Büffelmilch wird zentrifugiert und die obere Fettschicht wird entfernt;
- besagte neue Menge an Milch mit entzogenem Fett und besagte einzige endgültige Lösung werden in einem weiteren Behälter vermischt, um um Lactoferrin selbstbereicherte Büffelmilch zu erhalten;
- eine definierte Menge Fett wird der besagten um Lactoferrin selbstbereicherten Büffelmilch zugegeben,
um ein nahrhaftes Getränk, wie das beschriebene zu erhalten, mit dem zusätzlichen Merkmal eines Fettgehalts, der niedriger als der der Büffelmilch ist.

4. Verfahren zur Extraktion von Lactoferrin aus Büffelmilch, gemäß einem oder mehreren vorherigen Ansprüchen, das **dadurch gekennzeichnet ist, dass** die obengenannte Anzahl an Dialysezyklen N gleich 2 ist.

5. Verfahren zur Extraktion von Lactoferrin aus Büffelmilch, gemäß einem oder mehreren vorherigen Ansprüchen, das **dadurch gekennzeichnet ist, dass** die obengenannte definierte Menge an Büffelmilch gleich 1 Liter ist.

6. Verfahren zur Extraktion von Lactoferrin aus Büffelmilch, gemäß einem oder mehreren vorherigen Ansprüchen, das **dadurch gekennzeichnet ist, dass** die obengenannte definierte Menge an bidestilliertem Wasser gleich 50 Milliliter ist.

7. Verfahren zur Extraktion von Lactoferrin aus Büffelmilch, gemäß dem vorherigen Anspruch 1, das **dadurch gekennzeichnet ist, dass**:
- die obengenannte definierte Menge an Büffelmilch zuvor einem Prozess der Zentrifugation unterzogen und die obere Fettschicht entfernt wurde;
und durch die folgenden zusätzlichen Schritte gekennzeichnet ist:
- nachdem die obengenannte erste konzentrierte Lösung Lactoferrin gebildet wurde, wird der obengenannte Dialyseschlauch durch einen anderen neuen ersetzt, der eine gleiche Menge an Büffelmilch mit dem entfernten Fettgehalt enthält;
- eine Reihe von den vorherigen identischen Zyklen in endlicher Anzahl aufeinanderfolgen, bis man eine Lösung, die aus dem Verfahren mit einem Pegel N (Anzahl der Zyklen) an Lactoferrin- Konzentration gewonnen wurde, hat,
so dass aus den bemerkenswerten organoleptischen Eigenschaften und mit einem hohen Gehalt an Lactoferrin mit derselben antioxidativen Kraft von Büffelmilch ein nahrhaftes Getränk gewonnen wird, das daher in der Lage ist, die Immunabwehr zu stimulieren, die Eisenaufnahme durch den Darm zu erhöhen und eine Alternative zu Kuhmilch zu stellen, da es keine Erscheinungen der Nahrungsmittelunverträglichkeit erzeugt.

8. Verfahren zur Extraktion von Lactoferrin aus Büffelmilch, gemäß dem vorherigen Anspruch 7, das durch die folgenden zusätzlichen Schritte gekennzeichnet ist:
- eine neue vordefinierte Menge an Büffelmilch wird zentrifugiert und die obere Fettschicht wird entfernt;
- besagte neue Menge an Milch mit entzogenem Fett und besagte einzige endgültige Lösung werden in einem weiteren Behälter vermischt, um um Lactoferrin selbstbereicherte Büffelmilch zu erhalten;
- eine definierte Menge Fett wird der um Lactoferrin selbstbereicherten Büffelmilch zugegeben,
um ein nahrhaftes Getränk, wie das beschriebene zu erhalten, mit dem zusätzlichen Merkmal eines Fettgehalts, der niedriger als der von Büffelmilch ist.

9. Verfahren zur Extraktion von Lactoferrin aus Büffelmilch, gemäß einem oder mehreren vorherigen Ansprüchen, das **dadurch gekennzeichnet ist, dass** der obengenannte Dialyseschlauch eine semipermeable Membran aus Cellulose-Acetat hat.

10. Verfahren zur Extraktion von Lactoferrin aus Büffelmilch, gemäß einem oder mehreren vorherigen Ansprüchen 7 bis 9, das **dadurch gekennzeichnet ist, dass** die obengenannte Anzahl an Dialysezyklen N gleich 6 ist.

11. Verfahren zur Extraktion von Lactoferrin aus Büffelmilch, gemäß einem oder mehreren vorherigen Ansprüchen 7 bis 10, das **dadurch gekennzeichnet ist, dass** die obengenannte definierte Menge Büffelmilch gleich 100 Milliliter ist.

12. Verfahren zur Extraktion von Lactoferrin aus Büffelmilch, gemäß einem oder mehreren vorherigen Ansprüchen 7 bis 11, das **dadurch gekennzeichnet ist, dass** die obengenannte definierte Menge bidestilliertes Wasser gleich 100 Milliliter ist.

13. Verfahren zur Extraktion von Lactoferrin aus Büffelmilch, gemäß einem oder mehreren vorherigen Ansprüchen, das **dadurch gekennzeichnet ist, dass** die obengenannte definierte Temperatur gleich 4 °C und der obengenannte definierte Zeitintervall gleich 30 Minuten ist.

14. Verfahren zur Extraktion von Lactoferrin aus Büffelmilch, gemäß dem vorherigen Anspruch 3 oder auch dem vorherigen Anspruch 8, das **dadurch gekennzeichnet ist, dass** die besagte definierte Menge an Fett gleich 2-3% ist.

15. Verfahren zur Extraktion von Lactoferrin aus Büffelmilch, gemäß dem vorherigen Anspruch 3 oder auch dem vorherigen Anspruch 8, das **dadurch gekennzeichnet ist, dass** die besagte Zentrifugation bei 4000 Umdrehungen pro Minute für 10 Minuten erfolgt.

## Revendications

1. Procédé pour l'extraction de lactoferrine du lait de bufflonne, **caractérisé par** les étapes suivantes:
- dans un premier cycle, une certaine quantité de lait de bufflonne est introduite dans un tube de dialyse qui est en contact avec une certaine quantité d'eau bidistillée, à une certaine température et pendant un certain laps de temps;
- à la fin dudit laps de temps, une certaine quantité de lactoferrine et une certaine quantité de sels est diffusée par ladite quantité de lait de bufflonne à ladite quantité déterminée d'eau bidistillée, en formant à l'extérieur une première solution concentrée de lactoferrine.

2. Procédé pour l'extraction de lactoferrine du lait de bufflonne, selon la précédente revendication 1, **caractérisé par** les étapes supplémentaires suivantes:
- ladite première solution concentrée de lactoferrine est remplacée par une nouvelle et même quantité d'eau bidistillée;
- dans un second cycle, à une certaine température et pendant un certain laps de temps, une quantité supplémentaire de lactoferrine et de sels est diffusée par ladite quantité de lait de bufflonne à ladite quantité déterminée d'eau bidistillée, en formant à l'extérieur une deuxième solution concentrée de lactoferrine;
- une série de cycles identiques aux précédents se succèdent en nombre fini, jusqu'à obtenir N solutions concentrées de lactoferrine issues du procédé (une par cycle);
- lesdites N solutions concentrées de lactoferrine sont mélangées dans un récipient afin d'obtenir une solution finale unique, ayant une teneur en lactoferrine supérieure à la teneur initiale dans le lait de bufflonne,
de façon à obtenir une boisson nutritive, aux remarquables propriétés organoleptiques et ayant une haute teneur en lactoferrine, avec le même pouvoir antioxydant du lait de bufflonne; ladite boisson est donc à même de stimuler les défenses immunitaires, d'augmenter l'assimilation du fer par l'intestin et de fournir une alternative au lait de vache, car elle ne génère aucun phénomène d'intolérance alimentaire.

3. Procédé pour l'extraction de lactoferrine du lait de bufflonne, selon la précédente revendication 2, **caractérisé par** les étapes supplémentaires suivantes:
- une nouvelle quantité déterminée de lait de bufflonne est soumise à la centrifugation et la couche supérieure de graisse est éliminée;
- ladite nouvelle quantité de lait sans graisse et ladite solution finale unique sont mélangées dans un autre récipient afin d'obtenir du lait de bufflonne auto-enrichi en lactoferrine;
- une certaine quantité de graisse est ajoutée dans ledit lait de bufflonne auto-enrichi en lactoferrine,
de façon à obtenir une boisson nutritive comme celle décrite, ayant la caractéristique supplémentaire d'une teneur en graisse inférieure à celle du lait de bufflonne.

4. Procédé pour l'extraction de lactoferrine du lait de bufflonne, selon une ou plusieurs des précédentes revendications, **caractérisé par le fait que** ledit nombre de cycles de dialyse N est égal à 2.

5. Procédé pour l'extraction de lactoferrine du lait de bufflonne, selon une ou plusieurs des précédentes revendications, **caractérisé par le fait que** ladite quantité déterminée de lait de bufflonne est égale à 1 litre.

6. Procédé pour l'extraction de lactoferrine du lait de bufflonne, selon une ou plusieurs des précédentes revendications, **caractérisé par le fait que** ladite quantité déterminée d'eau bidistillée est égale à 50 millilitres.

7. Procédé pour l'extraction de lactoferrine du lait de bufflonne, selon la précédente revendication 1, **caractérisé par le fait que**:
- ladite quantité déterminée de lait de bufflonne a préalablement été soumise à un processus de centrifugation et la couche supérieure de graisse a été éliminée;
et **caractérisé par** les étapes supplémentaires suivantes:
- après la formation de ladite première solution concentrée de lactoferrine, ledit tube de dialyse est remplacé par un nouveau tube, contenant une même quantité de lait de bufflonne dont le contenu de graisse a été éliminé;
- une série de cycles identiques aux précédents se succèdent en nombre fini, jusqu'à obtenir une solution issue du procédé ayant un niveau N (nombre de cycles) de teneur en lactoferrine,
de façon à obtenir une boisson nutritive, aux remarquables propriétés organoleptiques et ayant une haute teneur en lactoferrine, avec le même pouvoir antioxydant du lait de bufflonne; ladite boisson est donc à même de stimuler les défenses immunitaires, d'augmenter l'assimilation du fer par l'intestin et de fournir une alternative au lait de vache, car elle ne génère aucun phénomène d'intolérance alimentaire.

8. Procédé pour l'extraction de lactoferrine du lait de bufflonne, selon la précédente revendication 7, **caractérisé par** les étapes supplémentaires suivantes:
- une nouvelle quantité déterminée de lait de bufflonne est soumise à la centrifugation et la couche supérieure de graisse est éliminée;
- ladite nouvelle quantité de lait sans graisse et ladite solution finale unique sont mélangées dans un autre récipient afin d'obtenir du lait de bufflonne auto-enrichi en lactoferrine;
- une certaine quantité de graisse est ajoutée dans ledit lait de bufflonne auto-enrichi en lactoferrine,
de façon à obtenir une boisson nutritive comme celle décrite, ayant la caractéristique supplémentaire d'une teneur en graisse inférieure à celle du lait de bufflonne.

9. Procédé pour l'extraction de lactoferrine du lait de bufflonne, selon une ou plusieurs des précédentes revendications, **caractérisé par le fait que** ledit tube de dialyse a une membrane semi-perméable en acétate de cellulose.

10. Procédé pour l'extraction de lactoferrine du lait de bufflonne, selon une ou plusieurs des précédentes revendications de 7 à 9, **caractérisé par le fait que** ledit nombre de cycles de dialyse N est égal à 6.

11. Procédé pour l'extraction de lactoferrine du lait de bufflonne, selon une ou plusieurs des précédentes revendications de 7 à 10, **caractérisé par le fait que** ladite quantité déterminée de lait de bufflonne est égale à 100 millilitres.

12. Procédé pour l'extraction de lactoferrine du lait de bufflonne, selon une ou plusieurs des précédentes revendications de 7 à 11, **caractérisé par le fait que** ladite quantité déterminée d'eau bidistillée est égale à 100 millilitres.

13. Procédé pour l'extraction de lactoferrine du lait de bufflonne, selon une ou plusieurs des précédentes revendications, **caractérisé par le fait que** ladite température déterminée est de 4°C et ledit laps de temps déterminé est de 30 minutes.

14. Procédé pour l'extraction de lactoferrine du lait de bufflonne, selon la précédente revendication 3 ou la précédente revendication 8, **caractérisé par le fait que** ladite quantité déterminée de graisse est de 2-3%.

15. Procédé pour l'extraction de lactoferrine du lait de bufflonne, selon la précédente revendication 3 ou la précédente revendication 8, **caractérisé par le fait que** ladite centrifugation est effectuée à 4000 tours par minute pendant 10 minutes.
